(19) Europäisches Patentamt
European Patent Office
Office européen des brevets

(11) **EP 2 438 034 B1**

(12) **FASCICULE DE BREVET EUROPEEN**

(45) Date de publication et mention
de la délivrance du brevet:
**11.10.2017 Bulletin 2017/41**

(21) Numéro de dépôt: **10727092.8**

(22) Date de dépôt: **04.05.2010**

(51) Int Cl.:
*C07C 17/25* (2006.01)     *C07C 21/18* (2006.01)

(86) Numéro de dépôt international:
**PCT/FR2010/050847**

(87) Numéro de publication internationale:
**WO 2010/139873 (09.12.2010 Gazette 2010/49)**

(54) **PROCÉDÉ DE PRÉPARATION DE COMPOSÉS FLUORES OLÉFINIQUES**

VERFAHREN ZUR HERSTELLUNG VON FLUOROLEFINVERBINDUNGEN

PROCESS FOR PREPARING FLUOROOLEFIN COMPOUNDS

(84) Etats contractants désignés:
**AL AT BE BG CH CY CZ DE DK EE ES FI FR GB
GR HR HU IE IS IT LI LT LU LV MC MK MT NL NO
PL PT RO SE SI SK SM TR**

(30) Priorité: **04.06.2009 FR 0953701**

(43) Date de publication de la demande:
**11.04.2012 Bulletin 2012/15**

(73) Titulaire: **Arkema France
92700 Colombes (FR)**

(72) Inventeurs:
• **GUILLET, Dominique
F-69390 Vernaison (FR)**

• **DEVIC, Michel
F-69110 Sainte Foy Les Lyon (FR)**

(74) Mandataire: **Dang, Doris
ARKEMA FRANCE
Département Propriété Industrielle
420, rue d'Estienne d'Orves
92705 Colombes Cedex (FR)**

(56) Documents cités:
**EP-A- 0 974 571     WO-A-2008/075017
US-A- 3 499 048     US-A- 3 579 595**

## Description

## DOMAINE DE L'INVENTION

L'invention a pour objet un procédé de préparation de composés fluorés oléfiniques. Elle concerne plus particulièrement un procédé de préparation des hydrofluoropropènes.

## ARRIERE-PLAN TECHNOLOGIQUE

Les hydrofluorocarbones (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

Le 1,2,3,3,3- pentafluoropropène (HFO-1225ye) est un intermédiaire de synthèse dans la fabrication du 2,3,3,3-tétrafluoro-1-propène (HFO-1234yf).

La plupart des procédés de fabrication des hydrofluorooléfines font appel à une réaction de déhydrohalogénation. Ainsi, le document WO 03/027051 décrit un procédé de fabrication de fluorooléfines de formule $CF_3CY=CX_nH_p$, dans laquelle X et Y représentent chacun un atome d'hydrogène ou d'halogène choisi parmi le fluor, le chlore, le brome ou l'iode ; n et p sont des nombres entiers et peuvent indépendamment prendre la valeur zéro, 1 ou 2 à condition que (n + p) = 2, comprenant la mise en contact d'un composé de formule $CF_3C(R^1_aR^2_b)C(R^3_cR^4_d)$ avec $R^1$, $R^2$, $R^3$ et $R^4$ représentant indépendamment un atome d'hydrogène ou d'halogène choisi parmi le fluor, le chlore, le brome ou l'iode à condition qu'au moins un de $R^1$, $R^2$, $R^3$ et $R^4$ est un atome d'halogène et qu'au moins un atome d'hydrogène et un atome d'halogène sont situés sur des atomes de carbone adjacent ; a et b peuvent indépendamment prendre la valeur zéro, 1 ou 2 à condition que (a + b) = 2 ; c et d peuvent indépendamment prendre la valeur zéro, 1, 2 ou 3 à condition que (c + d) = 3, avec au moins un hydroxyde de métal alcalin en présence d'un catalyseur de transfert phase.

Ce document enseigne à l'exemple 2, qu'en l'absence d'un catalyseur de transfert de phase, il n'y a pas de réaction lorsque le 1,1,1,3,3-pentafluoropropane (HFC-245fa) est mis en contact avec une solution aqueuse de 50 % en poids de hydroxyde de potassium (KOH) pendant 24 heures à température ambiante et sous pression.

Ce document suggère en outre une température de réaction compris entre -20°C et 80°C mais ne recommande pas l'emploi des hydroxydes d'alcalino-terreux du fait de leur solubilité limitée dans l'eau.

Le document WO 2008/075017 divulgue un procédé de déhydrohalogénation comprenant la mise en contact d'un hydro(halo)fluoroalcane avec une base éventuellement en présence d'un solvant. Il enseigne qu'en l'absence d'un solvant, l' hydro(halo)fluoroalcane peut être envoyé sur la base à chaud ou à l'état fondu.

Ce document illustre la réaction de déhydrofluoration du 1,1,1,2,3,3-hexafluoropropane (HFC-236ea) en 1,2,3,3,3-pentafluoropropène (HFO-1225ye) à 150°C en présence d'une solution aqueuse de 50 % en poids de KOH. En l'absence d'un catalyseur de transfert de phase, la conversion au bout de 3 heures et demie est de 57,8 % et la sélectivité en HFO-1225ye est de 52,4 % (essai 1). En présence d'un catalyseur de transfert de phase, cette conversion est atteinte au bout de 2,5 heures seulement et la sélectivité est pratiquement inchangée (essai 4). Comme indiqué au tableau 2 de ce document pour accroître la sélectivité en HFO-1225ye, il est nécessaire d'utiliser un solvant organique.

WO 2007/056194 décrit la préparation de HFO-1234yf par déhydrofluoration du 1,1,1,2,3-pentafluoropropane (HFC-245eb) soit avec une solution aqueuse de KOH soit en phase gazeuse en présence d'un catalyseur, notamment sur catalyseur à base de nickel, de carbone ou une combinaison de ceux-ci.

Dans le document WO 2008/030440 est décrit un mode de réalisation de déhydrofluoration du 1,1,1,2,3-pentafluoropropane à l'aide d'une solution aqueuse basique en présence d'un solvant non aqueux, non alcoolique dans lequel le HFC-245eb est partiellement miscible.

Le document Knunyants et al, Journal of the USSR Academy of Sciences, Chemistry Department, "reactions of fluoro-olefins", report 13., "catalytic hydrogénation of perfluoro-olefins", 1960, décrit de façon distincte diverses réactions chimiques sur des composes fluorés. Ce document décrit la déhydrofluoration du 1,1,1,2,3,3-hexafluoropropane (236ea) par passage au travers d'une suspension de KOH en poudre dans l'éther de dibutyle, pour produire du 1,2,3,3,3-pentafluoropropène-1 (HFO-1225ye) avec un rendement de 60 % seulement. Ce document décrit également la déhydrofluoration de 1,1,1,2,3-pentafluoropropane (HFC-245eb) en 2,3,3,3-tétrafluoro-1-propène (HFO-1234yf) par passage dans une suspension de KOH en poudre dans l'éther de dibutyle avec un rendement de 70 % seulement. Les documents US 3,499,048 et US 3,579,595 divulguent la préparation respectivement du 1,1-dichloro-2,3,3-tetrafluoropropène et du 1-bromo-1-chloro-2,3,3-trifluoropropène par mise en contact des propanes correspondants de départ avec de la chaux sodée solide à faible humidité (2% eau). Les rendements obtenus sont inférieurs à l'invention. Enfin, l'article de Sianesi Dario et al. dans Annali Di Chimica, Societa Chimica Italiana, Rome, IT, vol. 55 no.8-9, 1 janvier 1965 pages 850-861 décrit la déhydrofluoration du 1,1,1,2,3,3-hexafluoropropane à l'aide du KOH.

Les réactions de déhydrofluoration telles que décrites ci-dessus conduisent, outre au composé hydrofluorooléfinique recherché, à la formation de l'eau et de fluorure de potassium. Par ailleurs, la mise en oeuvre d'une telle réaction en continu n'est pas aisée à l'échelle industrielle car au moins trois phases (gazeuse, liquide et solide) sont mis en jeu et l'élimination du fluorure de potassium formé en quantité stoechiométrique pose problème.

La présente invention fournit un procédé de fabrication continu ou semi-continu d'un composé (hydro)fluorooléfinique permettant de remédier aux inconvénients précités. La réaction mise enjeu dans le procédé est une réaction gaz-solide.

La présente invention a donc pour objet un procédé de fabrication continu ou semi-continu d'un composé (hydro)fluorooléfinique comprenant (i) la mise en contact d'au moins un composé comprenant de deux à six atomes de carbone, d'au moins deux atomes de fluor et d'au moins un atome d'hydrogène, à la condition qu'au moins un atome d'hydrogène et un atome de fluor soient situés sur des atomes de carbone adjacent, avec un réactif solide comprenant de l'hydroxyde de calcium avec un taux d'humidité compris entre 7 à 20 % en poids. avec co-formation du fluorure de calcium, sous produit valorisable.

Le procédé selon la présente invention fournit de préférence un composé (hydro)fluorooléfinique comportant trois atomes de carbone, avantageusement un composé (hydro)fluorooléfinique représenté par la formule (I)

$$CF_3CY=CX_nH_p \qquad (I)$$

dans laquelle Y représente un atome d'hydrogène ou d'halogène choisi parmi le fluor, le chlore, le brome ou l'iode et X représente un atome d'halogène choisi parmi le fluor, le chlore, le brome ou l'iode ; n et p sont des nombres entiers et peuvent indépendamment prendre la valeur zéro, 1 ou 2 à condition que (n + p) = 2, en mettant en contact un composé de formule $CF_3CYRCR'X_nH_p$, dans laquelle X, Y, n et p ont la même signification que dans la formule (I) et R représente un atome de fluor lorsque R' représente un atome d'hydrogène ou R représente un atome d'hydrogène lorsque R' représente un atome de fluor, avec un réactif solide comprenant de l'hydroxyde de calcium.

La présente invention convient tout particulièrement à la fabrication d'un composé de formule (Ia)

$$CF_3\text{-}CF=CHZ \qquad (Ia)$$

dans laquelle Z représente un atome d'hydrogène ou fluor à partir d'un composé de formule $CF_3CFRCHR'Z$ dans laquelle Z a la même signification que dans la formule (Ia) et R représente un atome de fluor lorsque R' représente un atome d'hydrogène ou R représente un atome d'hydrogène lorsque R' représente un atome de fluor.

Ainsi, le 2,3,3,3-tetrafluoropropène peut être obtenu par déhydrofluoration du 1,2,3,3,3-pentafluoropropane avec un réactif solide comprenant de l'hydroxyde de calcium et/ou le 1,2,3,3,3-pentafluoropropène par déhydrofluoration du 1,1,1,2,3,3-hexafluoropropane avec un réactif solide comprenant de l'hydroxyde de calcium. Le 1,2,3,3,3-pentafluoropropène peut être sous la forme d'isomère cis et/ou trans.

La présente invention peut en outre être utilisée pour la fabrication du 1,3,3,3-tetrafluoropropène par déhydrofluoration du 1,1,3,3,3-pentafluoropropane avec un réactif solide comprenant de l'hydroxyde de calcium.

Dans le procédé selon la présente invention le réactif solide peut contenir essentiellement de l'hydroxyde de calcium ou peut comprendre de l'hydroxyde de calcium en majorité et d'autres hydroxydes métalliques comme NaOH, KOH, Mg $(OH)_2$ et $Ba(OH)_2$ .

On peut avantageusement utiliser comme réactif solide la chaux sodée (Soda lime) qui est de l'hydroxyde de calcium contenant quelques pour cent en poids de NaOH et de KOH.

La teneur en KOH dans le réactif solide est de préférence comprise entre 0,1 et 5 % en poids et avantageusement comprise entre 2 et 4 % en poids.

La teneur en NaOH dans le réactif solide est de préférence comprise entre 0,1 et 5 % en poids et avantageusement comprise entre 1 et 3 % en poids.

Le réactif solide, comprenant de l'hydroxyde de calcium, peut être utilisé sous forme de poudre ou de préférence sous forme de granulés et présentant avantageusement une porosité élevée. Une granulométrie de granulés comprise entre 1 et 10 mm est préférée.

Le taux d'humidité présent dans le réactif solide est compris entre 7 à 20 % en poids et plus particulièrement compris entre 10 et 15 % en poids.

A titre d'exemple de réactif solide, on peut citer la chaux sodée, notamment celle commercialisée par la société GRACE sous la marque SODASORB HP.

Le procédé selon la présente invention est de préférence mis en oeuvre à une température comprise entre 100 et 170° C, avantageusement comprise entre 120 et 150° C. Une température comprise entre 130 et 140°C est particulièrement préférée. La réaction de déhydrofluoration, selon le procédé de la présente invention, peut être mise en oeuvre à pression atmosphérique mais on préfère travailler à une pression supérieure à la pression atmosphérique, par exemple entre 1 et 10 bars absolu. Avantageusement, cette pression est comprise entre 5 et 10 bars absolu.

La réaction peut être mise en oeuvre selon les moyens habituels utilisés dans l'industrie pour effectuer une réaction entre un gaz et un solide, comme par exemple dans un réacteur en lit fluidisé avec le réactif solide en poudre fine ou bien dans un réacteur en lit fixe ou encore dans un réacteur tournant du type four à ciment avec le réactif solide en forme de granulé poreux.

Le procédé est de préférence mis en oeuvre en semi-continu dans un ou plusieurs réacteurs tubulaires en lit fixe fonctionnant alternativement, par exemple selon la technologie du traitement des gaz par des tamis moléculaires. Après réaction le réactif solide partiellement ou totalement transformé en fluorure de calcium est déchargé du réacteur. Le fluorure de calcium, après éventuelle séparation de l'hydroxyde de calcium non réagi, peut être utilisé comme matière première dans la fabrication d'acide fluorhydrique.

Le procédé selon la présente invention peut comprendre le recyclage des réactifs non réagis à l'étape de déhydrofluoration.

## PARTIE EXPERIMENTALE

### Exemple 1

Dans un réacteur tubulaire de 850 mm de long et de 43 mm de diamètre chauffé à 140 ° C par une résistance électrique, on charge 500 g de chaux sodée SIGMA ALDRICH de granulométrie 0,15mm. On fait traverser le réacteur par un courant gazeux de 0,41 mole / h de HFC-236ea et 0,2 mole/h d'un gaz inerte.

Les gaz sortant du réacteur traversent un réfrigérant à 10° C pour éliminer la vapeur d'eau puis sont condensés dans un piège cryogénique. Après 80 min de fonctionnement en continu, la conversion globale du HFC-236ea est de 98 % et la sélectivité globale en HFO-1225ye est de 99 %.

### Exemple 2

Dans le même appareillage que l'exemple 1 , on charge 575 g de chaux sodée SODASORB 4-8 REG HP (société GRACE),granulométrie 3-6 mm et dont la teneur en humidité est de 14-19 % en poids.

Le débit de réactif à l'entrée du réacteur est de 0,49 mole/h HFC-236ea et 0,17mole/h d'un gaz inerte.

Le tube est chauffé à 135° C et les gaz sortant du tube sont analysés et recueillis dans un piège cryogénique.

On observe une conversion de 100% de HFC-236ea pendant les 100 min de fonctionnement.

La sélectivité en HFO-1225ye est supérieure à 99 %

### Exemple 3

Dans le même appareillage que l'exemple 1, on charge 690 g de chaux sodée SODASORB 4-8 REG HP (société GRACE,granulométrie 3-6 mm et dont la teneur en humidité est de 14-19 % en poids.

On chauffe le tube à 120° C et on introduit un débit de 0,5 mole/h de HFC245eb.

Les gaz sortant du tube sont analysés et recueillis dans un piège cryogénique

Après 4 h 35 de fonctionnement en continu, la conversion de HFC-245eb est de 100% et la sélectivité en 1234yf est de 99 %.

## Revendications

1. Procédé de fabrication continu ou semi-continu d'un composé (hydro)fluorooléfinique comprenant (i) la mise en contact d'au moins un composé comprenant de deux à six atomes de carbone, d'au moins deux atomes de fluor et d'au moins un atome d'hydrogène, à la condition qu' au moins un atome d'hydrogène et un atome de fluor sont situés sur des atomes de carbone adjacent, avec un réactif solide comprenant de l'hydroxyde de calcium, **caractérisé en ce que** le taux d'humidité du réactif solide est compris entre 7 à 20 % en poids.

2. Procédé selon la revendication 1 **caractérisé en ce que** le composé (hydro)fluorooléfinique de formule (I)

$$CF_3CY=CX_nH_p \qquad (I)$$

dans laquelle Y représente un atome d'hydrogène ou d'halogène choisi parmi le fluor, le chlore, le brome ou l'iode et X représente un atome d'halogène choisi parmi le fluor, le chlore, le brome ou l'iode ; n et p sont des nombres entiers et peuvent indépendamment prendre la valeur zéro, 1 ou 2 à condition que (n + p) = 2, comprenant la mise en contact d' un composé de formule $CF_3CYRCR'X_nH_p$, dans laquelle X, Y, n et p ont la même signification que dans la formule (I) et R représente un atome de fluor lorsque R' représente un atome d'hydrogène ou R représente un atome d'hydrogène lorsque R' représente un atome de fluor, avec un réactif solide comprenant de l'hydroxyde de calcium.

3. Procédé selon la revendication 1 **caractérisé en ce que** le composé (hydro)fluorooléfinique est de formule (Ia)

$$CF_3\text{-}CF=CHZ \qquad (Ia)$$

dans laquelle Z représente un atome d'hydrogène ou fluor comprend la mise en contact d'un composé de formule $CF_3CFRCHR'Z$, dans laquelle Z a la même signification que dans la formule (Ia) et R représente un atome de fluor lorsque R' représente un atome d'hydrogène ou R représente un atome d'hydrogène lorsque R' représente un atome de fluor, avec un réactif solide comprenant de l'hydroxyde de calcium solide.

**4.** Procédé selon l'une quelconque des revendications précédentes **caractérisée en ce que** le 2,3,3,3-tetrafluoropropène est obtenu par la mise en contact du 1,2,3,3,3-pentafluoropropane avec un réactif solide comprenant de l'hydroxyde de calcium et/ou le 1,2,3,3,3-pentafluoropropène par la mise en contact du 1,1,1,2,3,3-hexafluoropropane avec un réactif solide comprenant de l'hydroxyde de calcium.

**5.** Procédé selon l'une quelconque des revendications précédentes **caractérisé en ce que** le réactif solide comprend entre 0,1 et 5 % en poids de KOH.

**6.** Procédé selon l'une quelconque des revendications précédentes **caractérisé en ce que** le réactif solide comprend entre 0,1 et 5 % en poids de NaOH.

**7.** Procédé selon l'une quelconque des revendications précédentes **caractérisé en ce qu'**il est mis en oeuvre à une température comprise entre 100 et 170°C.

**8.** Procédé selon l'une quelconque des revendications précédentes **caractérisé en ce qu'**il est mis en oeuvre à une pression comprise entre 1 et 10 bar absolu.

**9.** Procédé selon l'une quelconque des revendications précédentes **caractérisé en ce que** le réactif solide est sous forme de granulés de 1 à 10 mm.

**Patentansprüche**

**1.** Verfahren zur kontinuierlichen oder halbkontinuierlichen Herstellung einer (Hydro)Fluorolefin-Verbindung, umfassend (i) das Inkontaktbringen mindestens einer Verbindung, die zwei bis sechs Kohlenstoffatome, mindestens zwei Fluoratome und mindestens ein Wasserstoffatom umfasst, mit der Maßgabe, dass mindestens ein Wasserstoffatom und ein Fluoratom sich an benachbarten Kohlenstoffatomen befinden, mit einem festen Reaktanten, der Calciumhydroxid umfasst, **dadurch gekennzeichnet, dass** der Feuchtigkeitsgehalt des festen Reaktanten zwischen 7 und 20 Gew.-% beträgt.

**2.** Verfahren nach Anspruch 1, **dadurch gekennzeichnet, dass** die (Hydro)Fluorolefin-Verbindung der Formel (I)

$$CF_3CY=CX_nH_p \qquad (I),$$

worin Y ein Wasserstoff- oder ein aus Fluor, Chlor, Brom oder Iod ausgewähltes Halogenatom darstellt und X ein aus Fluor, Chlor, Brom oder Iod ausgewähltes Halogenatom darstellt; n und p ganze Zahlen sind und unabhängig den Wert null, 1 oder 2 annehmen können, mit der Maßgabe, dass (n + p) = 2, umfassend das Inkontaktbringen einer Verbindung der Formel $CF_3CYRCR'X_nH_p$, worin X, Y, n und p die gleiche Bedeutung wie in Formel (I) haben und R ein Fluoratom darstellt, wenn R' ein Wasserstoffatom darstellt, oder R ein Wasserstoffatom darstellt, wenn R' ein Fluoratom darstellt, mit einem festen Reaktanten, der Calciumhydroxid umfasst.

**3.** Verfahren nach Anspruch 1, **dadurch gekennzeichnet, dass** die (Hydro)Fluorolefin-Verbindung der Formel (Ia) entspricht

$$CF_3-CF=CHZ \qquad (Ia),$$

worin Z ein Wasserstoff- oder Fluoratom darstellt, umfasst das Inkontaktbringen einer Verbindung der Formel $CF_3CFRCHR'Z$, worin Z die gleiche Bedeutung wie in Formel (Ia) hat und R ein Fluoratom darstellt, wenn R' ein Wasserstoffatom darstellt, oder R ein Wasserstoffatom darstellt, wenn R' ein Fluoratom darstellt, mit einem festen Reaktanten, der festes Calciumhydroxid umfasst.

**4.** Verfahren nach einem der vorhergehenden Ansprüche, **dadurch gekennzeichnet, dass** 2,3,3,3-Tetrafluorpropen mittels Inkontaktbringen von 1,2,3,3,3-Pentafluorpropan mit einem festen Reaktanten, der Calciumhydroxid umfasst, und/oder 1,2,3,3,3-Pentafluorpropen mittels Inkontaktbringen von 1,1,1,2,3,3-Hexafluorpropan mit einem festen Reaktanten, der Calciumhydroxid umfasst, erhalten wird.

**5.** Verfahren nach einem der vorhergehenden Ansprüche, **dadurch gekennzeichnet, dass** der feste Reaktant zwischen 0,1 und 5 Gew.-% KOH umfasst.

**6.** Verfahren nach einem der vorhergehenden Ansprüche, **dadurch gekennzeichnet, dass** der feste Reaktant zwischen 0,1 und 5 Gew.-% NaOH umfasst.

**7.** Verfahren nach einem der vorhergehenden Ansprüche, **dadurch gekennzeichnet, dass** es bei einer Temperatur zwischen 100 und 170°C durchgeführt wird.

**8.** Verfahren nach einem der vorhergehenden Ansprüche, **dadurch gekennzeichnet, dass** es bei einem Druck zwischen 1 und 10 bar absolut durchgeführt wird.

**9.** Verfahren nach einem der vorhergehenden Ansprüche, **dadurch gekennzeichnet, dass** der feste Reaktant in Form von Granulaten von 1 bis 10 mm vorliegt.

## Claims

1. Process for the continuous or semicontinuous manufacture of a (hydro)fluoroolefin compound, comprising (i) bringing at least one compound comprising from 2 to 6 carbon atoms, at least two fluorine atoms and at least one hydrogen atom, provided that at least one hydrogen atom and one fluorine atom are situated on adjacent carbon atoms, into contact with a solid reactant comprising calcium hydroxide, **characterized in that** the moisture content of the solid reactant is between 7 and 20% by weight.

2. Process according to Claim 1, **characterized in that** the (hydro)fluoroolefin compound of formula (I)

$$CF_3CY{=}CX_nH_p \qquad (I)$$

in which Y represents a hydrogen or halogen atom chosen from fluorine, chlorine, bromine or iodine and X represents a halogen atom chosen from fluorine, chlorine, bromine or iodine; and n and p are integers and can independently take the value zero, 1 or 2, provided that $(n + p) = 2$, which comprises bringing a compound of formula $CF_3CYRCR'X_nH_p$, in which X, Y, n and p have the same meanings as in the formula (I) and R represents a fluorine atom when R' represents a hydrogen atom or R represents a hydrogen atom when R' represents a fluorine atom, into contact with a solid reactant comprising calcium hydroxide.

3. Process according to Claim 1, **characterized in that** the (hydro)fluoroolefin compound is of formula (Ia)

$$CF_3{-}CF{=}CHZ \qquad (Ia)$$

in which Z represents a hydrogen or fluorine atom, comprises bringing a compound of formula $CF_3CFRCHR'Z$, in which Z has the same meaning as in the formula (Ia) and R represents a fluorine atom when R' represents a hydrogen atom or R represents a hydrogen atom when R' represents a fluorine atom, into contact with a solid reactant comprising solid calcium hydroxide.

4. Process according to any one of the preceding claims, **characterized in that** 2,3,3,3-tetra-fluoropropene is obtained by bringing 1,2,3,3,3-pentafluoropropane into contact with a solid reactant comprising calcium hydroxide and/or 1,2,3,3,3-penta-fluoropropene is obtained by bringing 1,1,1,2,3,3-hexafluoropropane into contact with a solid reactant comprising calcium hydroxide.

5. Process according to any one of the preceding claims, **characterized in that** the solid reactant comprises between 0.1 and 5% by weight of KOH.

6. Process according to any one of the preceding claims, **characterized in that** the solid reactant comprises between 0.1 and 5% by weight of NaOH.

7. Process according to any one of the preceding claims, **characterized in that** it is carried out at a temperature of between 100 and 170°C.

8. Process according to any one of the preceding claims, **characterized in that** it is carried out at a pressure of between 1 and 10 bar absolute.

9. Process according to any one of the preceding claims, **characterized in that** the solid reactant is in the form of granules of from 1 to 10 mm.

## RÉFÉRENCES CITÉES DANS LA DESCRIPTION

**Documents brevets cités dans la description**

- WO 03027051 A
- WO 2008075017 A
- WO 2007056194 A
- WO 2008030440 A
- US 3499048 A
- US 3579595 A

**Littérature non-brevet citée dans la description**

- **KNUNYANTS et al.** reactions of fluoro-olefins'', report 13., ''catalytic hydrogénation of perfluoro-olefins. *Journal of the USSR Academy of Sciences, Chemistry Department,* 1960
- **SIANESI DARIO et al.** *Annali Di Chimica, Societa Chimica Italiana,* 01 Janvier 1965, vol. 55 (8-9), 850-861